# EUROPEAN PATENT APPLICATION

(11) **EP 4 632 384 A1**
(43) Date of publication of application: **15.10.2025**
(21) Application number: 23900574.7
(22) Date of filing: 01.12.2023
(51) Int. Cl.: G01N 33/68, C07K 14/46, G01N 33/53

(54) **METHOD FOR DETERMINING COGNITIVE DYSFUNCTION STAGE**

(30) Priority: 05.12.2022 JP 2022194368
(71) Applicant: AlzMed, Inc., Tokyo 113-8485 (JP)
(72) Inventor: SHIRAO Tomoaki, Tokyo 113-8485 (JP); SEKINO Yuko, Tokyo 113-8485 (JP); SHOJI Mikio, Takasaki-shi, Gunma 370-0046 (JP)
(74) Representative: D Young & Co LLP
(86) International application number: PCT/JP2023/043089
(87) International publication number: WO 2024/122459

(57) **Abstract**

An object is to determine a cognitive dysfunction stage of a subject early. The present disclosure provides a method for determining a cognitive dysfunction stage of a subject comprising: a step of measuring a level or amount of at least one of drebrin A or drebrin A-derived molecules in a biological sample collected from the subject; and a step of determining a cognitive dysfunction stage of the subject based on the level or amount.

## Description

### Technical Field

The present disclosure relates to a method for determining a cognitive dysfunction stage of a subject. More particularly, the present disclosure relates to a method for determining a cognitive dysfunction stage based on the level or amount of at least one of drebrin A or drebrin A-derived molecules.

### Background Art

It is considered that Alzheimer's disease, that is, a principal cause of dementia, is an irreversible progressive brain disease, and is caused by dysfunction of synapses in the brain. Increase of the number of Alzheimer's disease patients has become great social interest in recent years, and there are demands for establishment of an early diagnosis method and a therapeutic drug therefor. It is known that senile plaques are observed in the brain of an Alzheimer's disease patient after death, and that these are an aggregate of "amyloid β protein" (amyloid plaques). The deposit of the amyloid β protein is the earliest lesion that can be pathologically confirmed, and it has been reported that amyloid β protein aggregates to directly show nerve cell toxicity. It has been currently widely accepted, through genetic analysis of familial Alzheimer's disease patients, that abnormality in production and accumulation of amyloid β protein is widely related to the onset of Alzheimer's disease. This is designated as the amyloid cascade hypothesis. In this manner, it has been widely accepted, through a large number of studies, that amyloid β protein is the principal cause of Alzheimer's disease. Besides, it has been found that the accumulation of amyloid β protein in the brain has started 20 or more years before the onset.

The synaptic dysfunction observed in cognitive dysfunctions such as Alzheimer's disease may be caused also due to decreased blood flow in the brain (as in depression or the like), brain hemorrhage, cerebral infarction, drug dependence, and the like, and in addition to Alzheimer's disease dementia, dementias caused by mitochondrial genetic disease, and diabetes are also known. The diagnosis of Alzheimer's disease can be confirmed only by performing pathological anatomy after the death, and during lifetime, the diagnosis is performed by a question test or an imaging test. In this case, the diagnosis is performed by a question test or the like after subjective symptom such as severe forgetfulness has been felt, and objective diagnosis cannot be performed. Besides, a question test or an imaging test can be used for definitive diagnosis of the late stage of dementia, but cannot be used for early diagnosis for mild cognitive impairment (MCI), and thus, early treatment cannot be started actually.

### Summary of the Invention

### Means to Solve the Object

Therefore, the present disclosure provides a technique for easily and accurately determining a cognitive dysfunction stage of a subject.

Accordingly, the present disclosure provides the followings:
(Item 1) A method for determining a cognitive dysfunction stage of a subject, comprising:
   a step of measuring a level or amount of at least one of drebrin or drebrin-derived molecules in a biological sample collected from the subject; and
   a step of determining a cognitive dysfunction stage of the subject based on the level or amount.
(Item A1) A method for determining a cognitive dysfunction stage of a subject, comprising:
   a step of measuring a level or amount of at least one of drebrin A or drebrin A-derived molecules in a biological sample collected from the subject; and
   a step of determining a cognitive dysfunction stage of the subject based on the level or amount.
(Item A2) The method according to any one of the above-described items, wherein the drebrin A or drebrin A-derived molecule comprises drebrin A-related protein (DARP).
(Item A3) The method according to any one of the above-described items, wherein the DARP comprises an amino acid sequence of at least positions 221 to 353 in SEQ ID NO: 1 and/or at least a part of the amino acid sequence.
(Item A4) The method according to any one of the above-described items, further comprising a step of measuring levels or amounts of at least one of amyloid βs, and/or at least one of phosphorylated taus in the biological sample.
(Item A5) The method according to any one of the above-described items, comprising a step of measuring levels or amounts of at least one of amyloid βs and at least one of phosphorylated taus in the biological sample.
(Item A6) The method according to any one of the above-described items, wherein the biological sample is selected from the group consisting of cerebrospinal fluid, blood, urine, saliva, and lacrimal fluid.
(Item A6a) The method according to any one of the above-described items, wherein the biological sample is cerebrospinal fluid.
(Item A6b) The method according to any one of the above-described items, wherein the measuring comprises measuring a sample obtained by pretreating the biological sample.
(Item A7) The method according to any one of the above-described items, wherein the subject is determined to have mild cognitive impairment (MCI) of Alzheimer's disease when the level or amount of at least one of drebrin A or drebrin A-derived molecules is an abnormal value.
(Item A9) The method according to any one of the above-described items, wherein the level or amount of at least one of amyloid βs is an abnormal value.
(Item A10) The method according to any one of the above-described items, wherein the subject is determined to be cognitively unimpaired (CU) in Alzheimer's disease when the biological sample is cerebrospinal fluid, and the level or amount of at least one of drebrin A or drebrin A-derived molecules is an abnormal value, and the level or amount of at least one of amyloid βs is a standard value.
(Item A10a) The method according to any one of the above-described items, wherein the drebrin A or drebrin A-derived molecule is A-DARP.
(Item A11) The method according to any one of the above-described items, wherein the subject is determined to be cognitively unimpaired (CU) in Alzheimer's disease when the biological sample is cerebrospinal fluid, and the level or amount of at least one of drebrin A or drebrin A-derived molecules is a standard value, and 1) when the level or amount of at least one of phosphorylated taus is an abnormal value, or 2) when the level or amount of at least one of amyloid βs is an abnormal value.
(Item A11a) The method according to any one of the above-described items, wherein the drebrin A or drebrin A-derived molecule is A-DARP.
(Item A12) The method according to any one of the above-described items, wherein the subject is determined to be cognitively unimpaired (CU) in Alzheimer's disease when the biological sample is cerebrospinal fluid, and the level or amount of at least one of drebrin A or drebrin A-derived molecules is a standard value, the level or amount of at least one of phosphorylated taus is an abnormal value, and the level or amount of at least one of amyloid βs is a standard value.
(Item A12a) The method according to any one of the above-described items, wherein the drebrin A or drebrin A-derived molecule is A-DARP.
(Item A13) The method according to any one of the above-described items, wherein the subject is determined to have no brain disorder when all the levels or amounts of at least one of drebrin A or drebrin A-derived molecules, at least one of amyloid βs, and at least one of phosphorylated taus are standard values.
(Item A13a) The method according to any one of the above-described items, wherein the biological sample is cerebrospinal fluid.
(Item A14) The method according to any one of the above-described items, wherein the subject is determined to have dementia of Alzheimer's disease when the level or amount of at least one of drebrin A or drebrin A-derived molecules is a standard value, the level or amount of at least one of phosphorylated taus is an abnormal value, and the level or amount of at least one of amyloid βs is an abnormal value.
(Item A15) The method according to any one of the above-described items, wherein when the biological sample is cerebrospinal fluid, an abnormal value of the level or amount of at least one of drebrin A or drebrin A-derived molecules is about 100 pg/ml or more.
(Item A15a) The method according to any one of the above-described items, wherein the biological sample is cerebrospinal fluid.
(Item A16) The method according to any one of the above-described items, wherein when the biological sample is cerebrospinal fluid, an abnormal value of the level or amount of at least one of the amyloid βs is about 900 pg/ml or less.
(Item A17) The method according to any one of the above-described items, wherein when the biological sample is cerebrospinal fluid, an abnormal value of the level or amount of at least one of the phosphorylated taus is about 50 pg/ml or more.
(Item A18) The method according to any one of the above-described items, wherein the level or amount of at least one of drebrin A or drebrin A-derived molecules is measured by using an antibody recognizing a drebrin A-specific epitope.
(Item AA1) A method for determining a cognitive dysfunction stage of a subject, comprising:
   a step of determining a cognitive dysfunction stage based on a level or amount of at least one of components of a biological sample collected from the subject,
   wherein (a) the subject is determined to have mild cognitive impairment (MCI) of Alzheimer's disease when the level or amount of at least one of drebrin A or drebrin A-derived molecules is an abnormal value in the biological sample collected from the subject,
   (b) the subject is determined to be cognitively unimpaired (CU) in Alzheimer's disease when the level or amount of at least one of drebrin A or drebrin A-derived molecules is an abnormal value, and the level or amount of at least one of amyloid βs is a standard value in the biological sample collected from the subject,
   (c) the subject is determined to be cognitively unimpaired (CU) in Alzheimer's disease when the biological sample is cerebrospinal fluid, and the level or amount of at least one of drebrin A or drebrin A-derived molecules is a standard value, and 1) when the level or amount of at least one of phosphorylated taus is an abnormal value, or 2) when the level or amount of at least one of amyloid βs is an abnormal value in the biological sample collected from the subject,
   (d) the subject is determined to be cognitively unimpaired (CU) in Alzheimer's disease when the biological sample is cerebrospinal fluid, and the level or amount of at least one of drebrin A or drebrin A-derived molecules is a standard value, the level or amount of at least one of phosphorylated taus is an abnormal value, and the level or amount of at least one of amyloid βs is a standard value in the biological sample collected from the subject,
   (e) the subject is determined to have no brain disorder when all the levels or amounts of at least one of drebrin A or drebrin A-derived molecules, at least one of amyloid βs, and at least one of phosphorylated taus are standard values in the biological sample collected from the subject, and
   (f) the subject is determined to have dementia of Alzheimer's disease when the level or amount of at least one of drebrin A or drebrin A-derived molecules is a standard value, the level or amount of at least one of phosphorylated taus is an abnormal value, and the level or amount of at least one of amyloid βs is an abnormal value in the biological sample collected from the subject.
(Item B1) An agent for determining a cognitive dysfunction stage of a subject based on a level or amount of at least one of drebrin A or drebrin A-derived molecules, the agent comprising an antibody recognizing a drebrin A-specific epitope.
(Item C1) A method in which a level or amount of at least one of drebrin A or drebrin A-derived molecules is used as an index for determining a cognitive dysfunction stage of a subject, comprising:
   a step of measuring the level or amount of at least one of drebrin A or drebrin A-derived molecules in a biological sample collected from the subject; and
   a step of using the level or amount as an index for determining a cognitive dysfunction stage of the subject.

In the present disclosure, it is intended that one or a plurality of the above-described features can be provided in another combination in addition to the above-described combinations. It is noted that other embodiments and advantages of the present disclosure will be recognized by those skilled in the art upon reading the following detailed description of the present disclosure if necessary.

It is noted that features and remarkable functions and effects of the present disclosure in addition to those described above will be apparent for those skilled in the art with reference to the following section of embodiments of the invention and the accompanying drawings.

### Effect of the Invention

When a determination method of the present disclosure is employed, determination of a cognitive dysfunction stage that has been conventionally dependent on an imaging test or the like can be early and simply performed, which enables prevention of the onset of and early treatment of such a disease.

### Brief Description of Drawings

[Figure 1] Figure 1 is a graph illustrating results of analysis, performed in one embodiment of the present disclosure, of the relation between A-DARP and a brain cognitive dysfunction stage obtained in all cases.
[Figure 2] Figure 2 is a graph illustrating results of analysis, performed in one embodiment of the present disclosure, of the relation between A-DARP and a brain cognitive dysfunction stage obtained with patients except for Alzheimer's disease patients, and patients with complications excluded.
[Figure 3] Figure 3 is a graph illustrating results of analysis, performed in one embodiment of the present disclosure, of the relation between Aβ42 in cerebrospinal fluid and a brain cognitive dysfunction stage.
[Figure 4] Figure 4 is a diagram illustrating results of determination of brain cognitive dysfunction stages, obtained in one embodiment of the present disclosure, in 22 cases in total of Alzheimer's disease patients with no complications and healthy persons.
[Figure 5] Figure 5 is a diagram illustrating results of determination of brain cognitive dysfunction stages, obtained in one embodiment of the present disclosure, in 31 cases in total of Alzheimer's disease patients and healthy persons.

### Mode of Carrying Out the Invention

The present disclosure will now be described with reference to best modes thereof. It should be understood that expression in a singular form includes the concept of a plural form thereof unless otherwise mentioned in the entire description herein. Accordingly, it should be understood that an article in the singular form (e.g., "a", "an", or "the" in English) includes the concept of the plural form thereof unless otherwise mentioned. It should be also understood that terms used herein are used in meanings usually used in this field unless otherwise mentioned. Accordingly, all the technical terms and scientific technical terms used herein have the same meanings as those generally understood by those skilled in the art to which the present disclosure belongs unless otherwise defined. In case of contradiction, the present specification (including definition) shall prevail.

Now, the definitions and/or basic technical contents of terms particularly used herein will be appropriately described.

Herein, the term "about" means ±10% of a subsequent numerical value.

Herein, the term "cognitive dysfunction" refers to a state in which some dysfunction is substantially occurring in brain function such as perception function, memory function, attention function, or executive function, and is not limited in the presence or absence of symptoms, and severity.

Herein, the term "drebrin A" refers to an adult isoform in a nerve cell out of drebrins that are actin-binding proteins expressing in a large amount in a nerve cell in the development process. The drebrins are involved in morphogenesis of a nerve cell, particularly formation of a neurite by changing properties of actin fiber, and are present entirely in the cell body and neurite in a nerve cell migrating during the development, but are present specifically in the spine structure (dendritic spine) in a mature nerve cell. The drebrin has two isoforms of embryonic type drebrin E and adult type drebrin A, and drebrin A present specifically to the dendritic spine of a mature nerve cell has a characteristic of expressing only in a nerve cell. Besides, it is known that drebrin in the dendritic spine is widely lost in dementias such as Alzheimer's disease.

Herein, the term "drebrin A-derived molecule" refers to a part or a fragment of drebrin A, or a molecule obtained by arbitrarily modifying the part or fragment.

Herein, the term "drebrin A-related protein (DARP)" refers to a fragment or a splice variant of drebrin A having at least a part of Ins2 coding sequence characteristic of drebrin A, or a dimer, an oligomer, or a polymer containing the fragment or splice variant.

Herein, the term "A-DARP" refers to a peptide comprising a peptide sequence of at least positions 221 to 353 of drebrin A (SEQ ID NO: 1), or a part of the peptide sequence.

Herein, the term "CDA" refers to A-DARP present in cerebrospinal fluid.

Herein, the term "PDA" refers to A-DARP present in blood.

Herein, the term "abnormal value", regarding a biomarker, refers to a value that is not a standard value, and is also referred to as a "non-standard value".

Herein, the term "standard value", regarding a biomarker, refers to a value shown in a normal state, and is a characteristic of the value itself, and hence, even when the value itself is a standard value, the subject may be normal in some cases, and may not be normal (in a sick state) in other cases.

### (Preferred Embodiments)

Preferred embodiments of the present disclosure will now be described. The embodiments provided below are provided for better understanding of the present disclosure, and the scope of the present disclosure should not be limited to the following description. Accordingly, it is obvious for those skilled in the art that modifications can be appropriately made within the scope of the present disclosure in consideration of the description given herein. Besides, one of the following embodiments of the present disclosure may be singly used, or a combination thereof may be used.

One aspect of the present disclosure provides a method for determining a cognitive dysfunction stage of a subject, comprising a step of measuring a level or amount of at least one of drebrin A or drebrin A-derived molecules in a biological sample collected from the subject, and a step of determining a cognitive dysfunction stage of the subject based on the level or amount. In the method of the present disclosure, a cognitive dysfunction stage can be determined only by measuring the level or amount of at least one of drebrin A or drebrin A-derived molecules in the biological sample, and hence the method is useful for prevention of onset and early treatment of cognitive dysfunction.

The present disclosure is based on finding that the level or amount of human drebrin A locally present in the brain (SEQ ID NO: 1: MAGVSFSGHRLELLAAYEEVIREESAADWALYTYEDGSDDLKLAASGEGGLQELSGHF ENQKVMYGFCSVKDSQAALPKYVLINWVGEDVPDARKCACASHVAKVAEFFQGVDVIV NASSVEDIDAGAIGQRLSNGLARLSSPVLHRLRLREDENAEPVGTTYQKTDAAVEMKR INREQFWEQAKKEEELRKEEERKKALDERLRFEQERMEQERQEQEERERRYREREQQI EEHRRKQQTLEAEEAKRRLKEQSIFGDHRDEEEETHMKKSESEVEEAAAIIAQRPDNP REFFKQQERVASASAGSCDVPSPFNHRPGRPYCPFIKASDSGPSSSSSSSSSPPRTPF PYITCHRTPNLSSSLPCSHLDSHRRMAPTPIPTRSPSDSSTASTPVAEQIERALDEVT SSQPPPLPPPPPPAQETQEPSPILDSEETRAAAPQAWAGPMEEPPQAQAPPRGPGSPA EDLMFMESAEQAVLAAPVEPATADATEIHDAADTIETDTATADTTVANNVPPAATSLI DLWPGNGEGASTLQGEPRAPTPPSGTEVTLAEVPLLDEVAPEPLLPAGEGCATLLNFD ELPEPPATFCDPEEVEGEPLAAPQTPTLPSALEELEQEQEPEPHLLTNGETTQKEGTQ ASEGYFSQSQEEEFAQSEELCAKAPPPVFYNKPPEIDITCWDADPVPEEEEGFEGGD), a fragment or splice variant of drebrin A, or a drebrin A-related protein (DARP) that is a dimer, an oligomer, or a polymer containing the fragment or splice variant varies depending on the cognitive dysfunction stage of a subject. Accordingly, in one embodiment of the present disclosure, the drebrin A or drebrin A-derived molecule may comprise a drebrin A-related protein (DARP). In the present disclosure, the DARP may be a fragment or splice variant having at least a part of Ins2 coding sequence RPYCPFIKASDSGPSSSSSSSSSPPRTPFPYITCHRTPNLSSSLPC (SEQ ID NO: 2) characteristic of drebrin A, or a dimer, an oligomer, or a polymer containing the fragment or splice variant. In one embodiment, the drebrin A or drebrin A-derived molecule such as a DARP may be singly used in the method of the present disclosure, or may be used in a combination of one or more molecules in the method of the present disclosure. Alternatively, a total concentration of the drebrin A or drebrin A-derived molecule such as a DARP may be measured to be used in the method of the present disclosure.

In one embodiment of the present disclosure, the DARP may comprise an amino acid sequence of at least positions 221 to 353 in the amino acid sequence of SEQ ID NO: 1, and in one embodiment, the DARP may comprise intact drebrin A at positions 1 to 795 of the amino acid sequence of SEQ ID NO: 1. A method for measuring intact drebrin A is known, and for example, a method described in Japanese unexamined Patent Application Publication No. 2016-40540 A is known.

In one embodiment of the present disclosure, the concentration of the drebrin A or drebrin A-derived molecule such as a DARP can be measured by any known method. In one embodiment of the present disclosure, the concentration of the drebrin A or drebrin A-derived molecule such as a DARP can be measured by, for example, a Western blotting method using an antibody recognizing the DARP, an ELISA method, an immunoprecipitation method or the like. The anti-DARP antibody may be either a monoclonal antibody or a polyclonal antibody as long as it is an antibody using a part of the DARP as an epitope, and one of such antibodies may be used, or two or more thereof may be used in combination. Besides, for distinction from drebrin E systemically present, the measurement is performed preferably with an antibody recognizing a drebrin A-specific epitope. In one embodiment, the present disclosure relates to an antibody recognizing a drebrin A-specific epitope for use in the method of the present disclosure. The term "drebrin A-specific epitope" means an epitope that is not present in drebrin E but present in drebrin A, and is preferably a region comprising at least a part of Ins2 coding sequence RPYCPFIKASDSGPSSSSSSSSSPPRTPFPYITCHRTPNLSSSLPC (SEQ ID NO: 2), and/or a specific three-dimensional structure formed for drebrin A to comprise the Ins2 coding sequence.

When the Western blotting method or the ELISA method is performed, a method in which an anti-DARP antibody is used as a primary antibody for performing detection with a labeled secondary antibody recognizing the primary antibody may be preferably employed. For example, when the primary antibody is a rabbit antibody, a labeled anti-rabbit IgG antibody may be used as the secondary antibody, and when the primary antibody is a mouse antibody, a labeled antimouse IgG antibody may be used.

Anti-DARP antibodies usable in the measurement in the present invention are known, and have been already commercially available. A specific example includes a DAS2 antibody described in THE JOURNAL OF COMPARATIVE NEUROLOGY 505: 352-362 (2007).

Examples of a labeling substance used in the labeled secondary antibody include an enzyme, a radioisotope, a fluorescent substance, a luminescent substance, and a gold colloid. Among these, an enzyme is preferred from the viewpoints of sensitivity and operation easiness, and horseradish peroxidase (HRP), alkaline phosphatase (AP), glucose oxidase (GOD) and the like are more preferred. When HRP is used as the labeling substance, TMB (3,3',5,5'-tetramethylbenzidine) may be used as a substrate, when AP is used, AMPPD (3-(2'-spiroadamantane)-4-methoxy-4-(3''-phosphoryloxy)phenyl-1,2-dioxetane disodium salt), 9-(4-chlorophenylthiophosphoryloxymethylidene)-10-methylacridan disodium salt, or a substrate for chemiluminescence may be used as a substrate. Alternatively, as the labeling substance, a fluorescent dye such as FITC (fluoresceinisothiocyanate), or rhodamine can be used.

A method for detecting/quantitatively determining drebrin A or drebrin A-derived molecule such as a DARP varies depending on the labeling method, and can be performed by a method known to and usually employed by those skilled in the art. For example, when HRP, AP, GOD, or the like is used as the labeling substance, a chromogenic substrate or a luminescent substrate is added for measuring an absorbance or luminescence intensity, and thus, a measurement target substance can be quantitatively determined. Alternatively, when a fluorescent substance is used as the labeling substance, the measurement target substance can be quantitatively determined by measuring the fluorescence intensity. When a radioisotope is used as the labeling substance, the measurement target substance can be quantitatively determined by measuring radioactivity. When a gold colloid is used as the labeling substance, the measurement target substance can be quantitatively determined by measuring an absorbance. In the quantitative determination, a calibration curve (standard curve) may be previously created by using a sample having a known concentration of drebrin A or drebrin A-derived molecule such as a DARP, and a measured value may be compared with the calibration curve to calculate the concentration of drebrin A or drebrin A-derived molecule such as a DARP in a sample to be used in comparison in the method of the present disclosure. Alternatively, without calculating the concentration of the drebrin A or drebrin A-derived molecule such as a DARP, the absorbance, the radioactivity, or the luminescence intensity may be directly used in the comparison in the method of the present disclosure. In one embodiment, for evaluating (determining) whether or not the level or concentration of drebrin A or drebrin A-derived molecule such as a DARP (for example, the concentration of the DARP calculated as described above, or the absorbance, radioactivity, or luminescence intensity) in a subject is higher or lower than the level or concentration of drebrin A or drebrin A-derived molecule such as a DARP in a control, an arbitrary threshold value (cut-off value) may be set, and examples of such a threshold value include an average, an average + standard deviation (SD), an average + 2SD, an average + 3SD, an average - SD, an average - 2SD, an average - 3SD, a median, a median + SD, a median + 2SD, a median + 3SD, a median - SD, a median - 2SD, and a median - 3SD of the level or concentration of drebrin A or drebrin A-derived molecule such as a DARP in the control.

In one embodiment of the present disclosure, a biological sample means a body fluid collected from a subject or a control subject. In one embodiment, examples of the body fluid include cerebrospinal fluid, blood, urine, saliva, and lacrimal fluid, among which cerebrospinal fluid and blood are preferred. Blood includes serum, plasma, and the like. The biological sample may be directly supplied to the measurement of the level or concentration of drebrin A or drebrin A-derived molecule such as a DARP, but is preferably subjected to a pretreatment for increasing detection sensitivity for drebrin A or drebrin A-derived molecule such as a DARP before the measurement. A pretreatment method may be any method for increasing the detection sensitivity for a measurement target protein present in the biological sample, and an example includes immunoprecipitation using an antibody for detecting drebrin A or drebrin A-derived molecule such as a DARP. Alternatively, when the biological sample is treated with a protein A/G bead, and a fraction adsorbed onto the bead is used in the method of the present disclosure, the detection sensitivity for drebrin A or drebrin A-derived molecule such as a DARP can be increased.

In one embodiment of the present disclosure, the method of the present disclosure may further comprise a step of measuring levels or amounts of at least one of amyloid βs, and/or at least one of phosphorylated taus in the biological sample. As described above, in the method of the present disclosure, the cognitive dysfunction stage of a subject can be determined by measuring the level or amount of at least one of drebrin A or drebrin A-derived molecules in a biological sample collected from the subject, and by measuring, in addition to the level or amount of at least one of drebrin A or drebrin A-derived molecules, the levels or amounts of at least one of amyloid βs and/or at least one of phosphorylated taus, the accuracy of determining the cognitive dysfunction stage can be further increased, and a large contribution can be made to stratification of target patients for discovery of drugs for Alzheimer's disease, and determination of the effect of therapeutic drugs. Diabetes doubles the risk of the onset of dementia caused by Altheimer's disease, and if the CU stage or the mild cognitive impairment stage can be determined, diabetes can be appropriately controlled before the onset for controlling the number of patients. Besides, a large number of risk factors of dementia are known in addition to diabetes, and the onset risk of dementia can be reduced by controlling the factors before the onset.

In one embodiment of the present disclosure, the method of the present disclosure can determine the cognitive dysfunction stage as classifications of, for example, dementia of Alzheimer's disease, mild cognitive impairment (MCI) of Alzheimer's disease, cognitively unimpaired (CU) in Alzheimer's disease, and a disease except for Alzheimer's disease, and the stage classifications are not limited to these. For example, the method of the present disclosure can determine, as the cognitive dysfunction, the stages of Alzheimer's disease, vascular dementia, dementia with Lewy bodies, Parkinson's disease, corticobasal degeneration, amyotrophic lateral sclerosis, spinocerebellar degeneration, brain hemorrhage, cerebral infarction, brain tumor, and the like.

In one embodiment of the present disclosure, the subject of the method of the present disclosure can be a subject that has developed cognitive dysfunction, a subject that has not developed cognitive dysfunction, and/or a subject unknown whether or not he/she has developed cognitive dysfunction.

In one embodiment of the present disclosure, the control for the comparison in the level or amount of at least one of drebrin A or drebrin A-derived molecules of the subject can be a healthy person that has no or little risk of onset of cognitive dysfunction, or has not developed cognitive dysfunction. Besides, the threshold value can be set based on the level or amount of at least one of drebrin A or drebrin A-derived molecules in such a healthy person.

In one embodiment of the present disclosure, the method of the present disclosure can determine a cognitive dysfunction stage based on the following criteria:

In one embodiment, the subject can be determined to have mild cognitive impairment (MCI) of Alzheimer's disease when the level or amount of at least one of drebrin A or drebrin A-derived molecules is an abnormal value.

In one embodiment, the subject can be determined to have mild cognitive impairment (MCI) of Alzheimer's disease when the level or amount of at least one of drebrin A or drebrin A-derived molecules is an abnormal value, and the level or amount of at least one of amyloid βs is an abnormal value.

In one embodiment, the subject can be determined to be cognitively unimpaired (CU) in Alzheimer's disease when the biological sample is cerebrospinal fluid, and the level or amount of at least one of drebrin A or drebrin A-derived molecules is an abnormal value, and the level or amount of at least one of amyloid βs is a standard value. In this case, A-DARP, and/or a molecule comprising A-DARP is preferably used as the drebrin A or drebrin A-derived molecule.

In one embodiment, the subject can be determined to be cognitively unimpaired (CU) in Alzheimer's disease when the biological sample is cerebrospinal fluid, and the level or amount of at least one of drebrin A or drebrin A-derived molecules is a standard value, and 1) when the level or amount of at least one of phosphorylated taus is an abnormal value, or 2) when the level or amount of at least one of amyloid βs is an abnormal value. In this case, A-DARP, and/or a molecule comprising A-DARP is preferably used as the drebrin A or drebrin A-derived molecule.

In one embodiment, the subject can be determined to be cognitively unimpaired (CU) in Alzheimer's disease when the biological sample is cerebrospinal fluid, and the level or amount of at least one of drebrin A or drebrin A-derived molecules is a standard value, the level or amount of at least one of phosphorylated taus is an abnormal value, and the level or amount of at least one of amyloid βs is a standard value. In this case, A-DARP is preferably used as the drebrin A or drebrin A-derived molecule.

In one embodiment, the subject can be determined to have no brain disorder when all the levels or amounts of at least one of drebrin A or drebrin A-derived molecules, at least one of amyloid βs, and at least one of phosphorylated taus are standard values.

In one embodiment, the subject can be determined to have dementia of Alzheimer's disease when the level or amount of at least one of drebrin A or drebrin A-derived molecules is a standard value, the level or amount of at least one of phosphorylated taus is an abnormal value, and the level or amount of at least one of amyloid βs is an abnormal value.

In one embodiment, the determination of a cognitive dysfunction stage of a subject can be performed in the method of the present disclosure based on the criteria shown in the following table:

**[Table 1]**

| Drebrin | Aβ42 | Phosphorylated Tau | Determination |
|---|---|---|---|
| Abnormal | - | - | MCI |
| Abnormal | Standard | - | CU |
| Standard | Abnormal | - | CU |
| Standard | - | Abnormal | CU |
| Standard | Standard | Abnormal | CU |
| Standard | Standard | Standard | Normal |
| Standard | Abnormal | Abnormal | Dementia |

In one embodiment of the present disclosure, standard values and abnormal values of the levels or amounts of the at least one of drebrin A or drebrin A-derived molecules, at least one of amyloid βs, and/or at least one of phosphorylated taus used in the determination of a cognitive dysfunction stage by the method of the present disclosure can be appropriately set in accordance with the type of the biological sample used in the determination, the type of DARP used in the determination, the type of stages to be classified by the determination, and the properties of the subject. For example, when the biological sample is cerebrospinal fluid, the abnormal value of the level or amount of at least one of drebrin A or drebrin A-derived molecules can be set to about 100 pg/ml or more.

In another embodiment, when the biological sample is cerebrospinal fluid, the abnormal value of the level or amount of at least one of amyloid βs can be set to about 900 pg/ml or less. In one embodiment, when the biological sample is cerebrospinal fluid, the abnormal value of the level or amount of at least one of phosphorylated taus can be set to about 50 pg/ml or more.

In one embodiment, when the biological sample is blood, and drebrin A or drebrin A-derived molecule are A-DARP and/or a molecule comprising A-DARP, the abnormal value of the level or amount of at least one of drebrin A or drebrin A-derived molecules can be set to about 2000 pg/ml or less. In another embodiment, when the biological sample is blood, and drebrin A or drebrin A-derived molecule are A-DARP and/or a molecule comprising A-DARP, the abnormal value of the level or amount of at least one of drebrin A or drebrin A-derived molecules can be set to about 1000 pg/ml or more, or about 400 pg/ml or less. The abnormal value of the level or amount of at least one of drebrin A or drebrin A-derived molecules may be different depending on the measurement method and the measurement target, and the term "abnormal value", regarding a biomarker, refers to a value that is not a standard value, and is also referred to as a "non-standard value".

Another aspect of the present disclosure provides an agent for determining a cognitive dysfunction stage of a subject based on a level or amount of at least one of drebrin A or drebrin A-derived molecules, the agent comprising an antibody recognizing a drebrin A-specific epitope.

Another aspect of the present disclosure provides a method in which a level or amount of at least one of drebrin A or drebrin A-derived molecules is used as an index for determining a cognitive dysfunction stage of a subject, comprising a step of measuring the level or amount of at least one of drebrin A or drebrin A-derived molecules in a biological sample collected from the subject; and a step of using the level or amount as an index for determining a cognitive dysfunction stage of the subject. In one embodiment, the embodiments described in the other sections herein can be appropriately selected to be used in this method.

### (General Technology)

Molecular biological methods, biochemical methods, and microbiological methods employed herein are known and usually employed in this field, and are described in, for example, Sambrook J. et al. (1989). Molecular Cloning: A Laboratory Manual, Cold Spring Harbor and its 3rd Ed.

(2001); Ausubel, F. M. (1987). Current Protocols in Molecular Biology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M. (1992). Short Protocols in Molecular Biology: A Compendium of Methods from Current Protocols in Molecular Biology, Greene Pub. Associates; Ausubel, F. M. (1995). Short Protocols in Molecular Biology: A Compendium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compendium of Methods from Current Protocols in Molecular Biology, Wiley, and annual updates; Sninsky, J. J. et al. (1999).

PCR Applications: Protocols for Functional Genomics, Academic Press, Bessatsu Jikken Igaku "Idenshi Donyu & Hatsugen Kaiseki Jikken Ho" (Experimental Medicine Separate Volume "Method for Gene Transfer & Expression Analysis", Yodosha Co., Ltd., 1997, and the like, relevant portions (that can be the entire contents) of which are incorporated herein by reference.

For DNA synthesis techniques and nucleic acid chemistry for producing an artificially synthesized gene, gene synthesis or fragment synthesis services such as GeneArt, GenScript, and Integrated DNA Technologies (IDT) can be employed, and in addition, these are described in, for example, Gait, M. J. (1985). Oligonucleotide Synthesis: A Practical Approach, IRL Press; Gait, M. J. (1990). Oligonucleotide Synthesis: A Practical Approach, IRL Press; Eckstein, F. (1991). Oligonucleotides and Analogues: A Practical Approach, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T. (1996). Bioconjugate Techniques, Academic Press, relevant portions of which are incorporated herein by reference.

Herein, the term "or" is used when "at least one or more of" items enumerated in the sentence. Herein, when the phrase "within a range" between "two values" is used, the range encompasses the two values themselves.

The entire contents of reference documents such as scientific documents, patents, and patent applications cited herein are incorporated herein by reference to the same extent as if these are specifically described.

The present disclosure has been described so far with reference to the preferred embodiments for easing understanding. Now, the present disclosure will be described by way of examples, and it is noted that the above description and the following examples are provided only for the purpose of exemplification not for the purpose of limiting the present disclosure. Accordingly, the scope of the present disclosure is not limited by neither the embodiments nor the examples specifically described herein but limited only by the appending claims.

### Examples

### 1. Detection of Phosphorylated Tau and Amyloid β42 in Cerebrospinal Fluid, and Detection of DARP in Blood and Cerebrospinal Fluid

Blood and cerebrospinal fluid were obtained from patients with no complications, phosphorylated tau and amyloid β42 were detected in the cerebrospinal fluid, and DARP was detected in the blood and the cerebrospinal fluid.

Concentrations of amyloid β40 and amyloid β42 in the cerebrospinal fluid were measured by sandwich ELISA using Human β Amyloid (1-40) ELISA Kit WakoII and Human/Rat β Amyloid (1-42) ELISA Kit WakoHigh-Sensitive (Wako Pure Chemical Industries Ltd., Osaka, Japan) according to the instruction of the manufacturer. Besides, total tau and phosphorylated tau in the cerebrospinal fluid were measured with INNOTEST hTAU Ag and PHOSPHO-TAU (181P) (FUJIREBIO Inc., Tokyo, Japan).

DARP was measured by sandwich ELISA using a combination of antibodies capable of specifically detecting drebrin A. Specifically, hDrebrin-A ELISA kit sold and distributed by the present Applicant (ALMED, INC.) was used.

### 2. Determination of Brain Cognitive Dysfunction Stage

The values of the phosphorylated tau, the amyloid β42, and the DARP of the patients with no complications obtained as described above were divided into a standard value and an abnormal value. As for the DARP, a threshold value was determined with respect to the A kit measured value CDA (corresponding to A-DARP) in cerebrospinal fluid for the division into a standard value and an abnormal value.

The results of the determination of MCI and dementia made by singly using A-DARP, and the determination of MCI and dementia made by using a combination with amyloid β and/or phosphorylated tau were as follows.

### (Example 1: Determination of Brain Cognitive Dysfunction Stage Using A-DARP in 50 Cases)

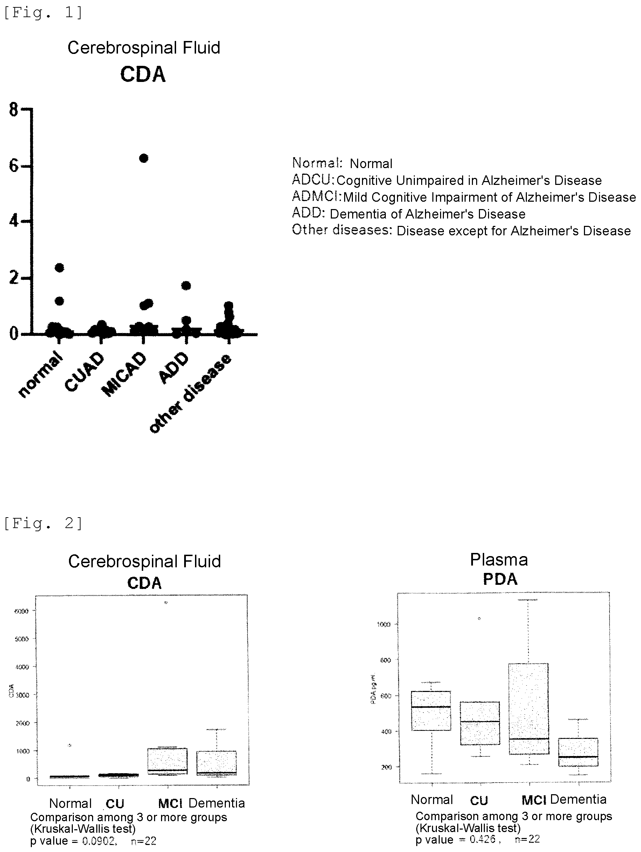

The results of analysis, by nonparametric Kruskal-Wallis test, of the relation between A-DARP and brain cognitive dysfunction stage in 50 cases are illustrated in Figure 1.

As illustrated in Figure 1, in all the cases, there was no significant difference in the CDA in the cerebrospinal fluid among a group of mild cognitive impairment of Alzheimer's disease (ADMCI), a group of dementia (ADD), and a normal group, and therefore, cases of complications were excluded from all the cases for examination in more detail.

### (Example 2: Determination of Brain Cognitive Dysfunction Stage Using A-DARP in Alzheimer's Disease Patients with No Complications)

The relation between the value measured with the hDrebrinA ELISA Kit and the brain cognitive dysfunction stage was analyzed with patients of diseases except for Alzheimer's disease and patients with complications excluded. The results are illustrated in Figure 2.

As illustrated in Figure 2, in the analysis performed with patients of diseases except for Alzheimer's disease and patients with complications excluded, it was found that the median of CDA of mild cognitive impairment was higher than in the other stages. Besides, it was found that when DARP (PDA) in blood was used, the medians of the PDA of the mild cognitive impairment group and the dementia group were lower than the median of the normal group. This result reveals that CDA and PDA can be used for determination of mild cognitive impairment.

### (Example 3: Determination of Brain Cognitive Dysfunction Stage Using Aβ42 in Cerebrospinal Fluid)

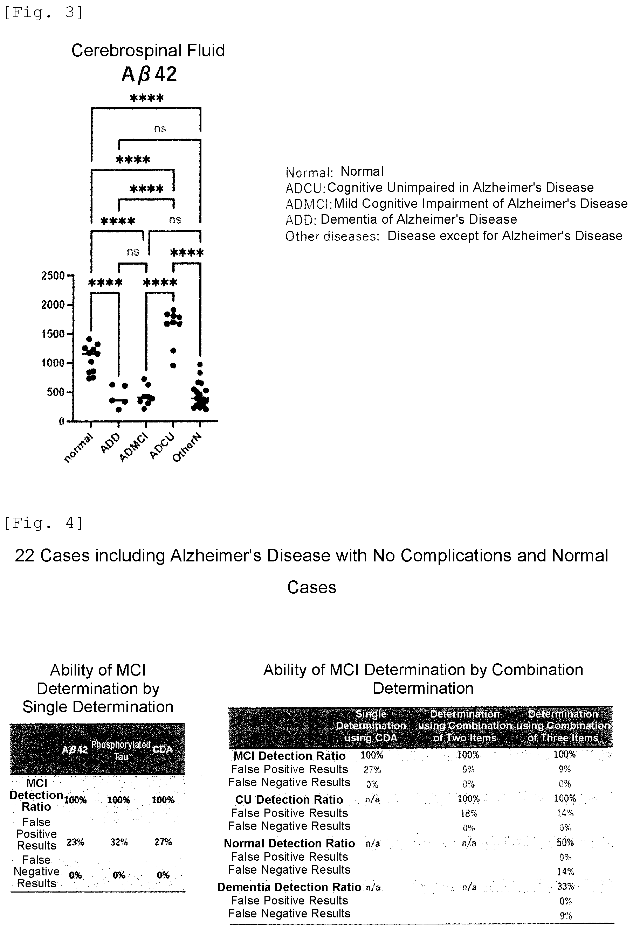

Subsequently, the relation between the measured value of Aβ42 in the cerebrospinal fluid and the brain cognitive dysfunction stage was analyzed in all the cases. The results are illustrated in Figure 3.

As illustrated in Figure 3, in all the cases, there was a significant difference in Aβ42 in the cerebrospinal fluid among the group of mild cognitive impairment of Alzheimer's disease (ADMCI), the group of dementia (ADD), and the normal group. This result reveals that Aβ42 in cerebrospinal fluid can be used for determination of MCI or dementia of Alzheimer's disease.

### (Example 4: Determination of Brain Cognitive Dysfunction Stage Using Combination of Amyloid β42, Phosphorylated Tau, and DARP)

Subsequently, determination of MCI was performed by singly using amyloid β42, phosphorylated tau, and A-DARP (CDA) in the cerebrospinal fluid of Alzheimer's disease patients, and determination of MCI was performed by using combinations thereof. Figure 4 illustrates the results of the determination of MCI obtained in 22 cases in total of Alzheimer's disease patients with no complications and healthy persons by singly using amyloid β42, phosphorylated tau, and A-DARP (COA), and the results of the determination of MCI obtained in the 22 cases in total of Alzheimer's disease patients and healthy persons by using combinations thereof.

As illustrated in Figure 4, it was found that when each of amyloid β42, phosphorylated tau, and A-DARP (CDA) in the cerebrospinal fluid is singly used, the determination of MCI can be performed, and that when a combination of A-DARP (CDA) with amyloid β42 or phosphorylated tau is used for the determination, the ratio of false positive and false negative results can be reduced.

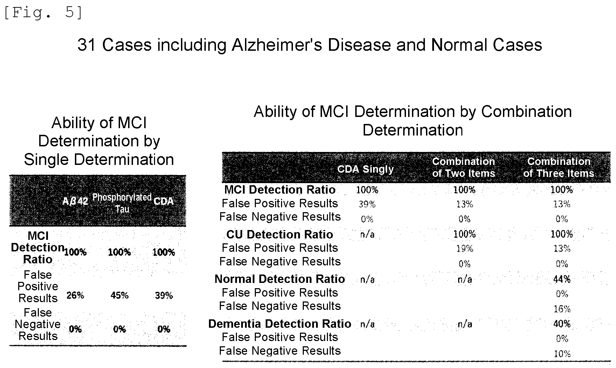

Figure 5 illustrates the results of determination of MCI obtained, in 31 cases in total including Alzheimer's disease patients and healthy persons, by singly using amyloid β42, phosphorylated tau, or A-DARP (CDA), and the results of determination of MCI obtained, in the 31 cases in total including Alzheimer's disease patients and healthy persons, by using combinations thereof.

As illustrated in Figure 5, it was found that also in the 31 cases, the determination of MCI can be performed by singly using amyloid β42, phosphorylated tau, or A-DARP (CDA), and that the ratio of false positive and false negative results can be reduced by performing the determination by using combinations of A-DARP (CDA) with amyloid β42, and phosphorylated tau.

### (Supplement)

The present disclosure has been exemplarily described so far by way of preferable embodiments of the present disclosure, and it is understood that the scope of the present disclosure should be interpreted only by the appending claims. It is understood that the contents of patents, patent applications and other documents cited herein are incorporated herein by reference in the same manner as if these are specifically described herein.

### Industrial Applicability

When a determination method of the present disclosure is employed, determination of a cognitive dysfunction stage that has been conventionally dependent on an imaging test or the like can be early and simply performed, which enables prevention of the onset of and early treatment of such a disease. Therefore, the method is expected to be applied in the medical and pharmaceutical fields.

### Sequence Listing Free Text

SEQ ID NO: 1: amino acid sequence of human drebrin A SEQ ID NO: 2: INS2 sequence

## Claims

1. A method for determining a cognitive dysfunction stage of a subject, comprising:
a step of measuring a level or amount of at least one of drebrin A or drebrin A-derived molecules in a biological sample collected from the subject; and
a step of determining a cognitive dysfunction stage of the subject based on the level or amount.

2. The method according to claim 1, wherein the drebrin A or drebrin A-derived molecule comprises drebrin A-related protein (DARP).

3. The method according to claim 2, wherein the DARP comprises an amino acid sequence of at least positions 221 to 353 in SEQ ID NO: 1, and/or at least a part of the amino acid sequence.

4. The method according to any one of claims 1 to 3, further comprising a step of measuring levels or amounts of at least one of amyloid βs, and/or at least one of phosphorylated taus in the biological sample.

5. The method according to claim 4, comprising a step of measuring levels or amounts of at least one of amyloid βs and at least one of phosphorylated taus in the biological sample.

6. The method according to any one of claims 1 to 5, wherein the biological sample is selected from the group consisting of cerebrospinal fluid, blood, urine, saliva, and lacrimal fluid.

7. The method according to any one of claims 1 to 6, wherein the biological sample is cerebrospinal fluid.

8. The method according to any one of claims 1 to 7, wherein the measuring comprises measuring a sample obtained by pretreating the biological sample.

9. The method according to any one of claims 1 to 8, wherein the subject is determined to have mild cognitive impairment (MCI) of Alzheimer's disease when the level or amount of at least one of drebrin A or drebrin A-derived molecules is an abnormal value.

10. The method according to claim 9, wherein the level or amount of at least one of amyloid βs is also an abnormal value.

11. The method according to any one of claims 1 to 8, wherein the subject is determined to be cognitively unimpaired (CU) in Alzheimer's disease when the biological sample is cerebrospinal fluid, and the level or amount of at least one of drebrin A or drebrin A-derived molecules is an abnormal value, and the level or amount of at least one of amyloid βs is a standard value.

12. The method according to any one of claims 1 to 8, wherein the subject is determined to be cognitively unimpaired (CU) in Alzheimer's disease when the biological sample is cerebrospinal fluid, and the level or amount of at least one of drebrin A or drebrin A-derived molecules is a standard value, and 1) when the level or amount of at least one of phosphorylated taus is an abnormal value, or 2) when the level or amount of at least one of amyloid βs is an abnormal value.

13. The method according to any one of claims 1 to 8, wherein the subject is determined to be cognitively unimpaired (CU) in Alzheimer's disease when the biological sample is cerebrospinal fluid, and the level or amount of at least one of drebrin A or drebrin A-derived molecules is a standard value, the level or amount of at least one of phosphorylated taus is an abnormal value, and the level or amount of at least one of amyloid βs is a standard value.

14. The method according to any one of claims 1 to 8, wherein the subject is determined to have no brain disorder when all the levels or amounts of at least one of drebrin A or drebrin A-derived molecules, at least one of amyloid βs, and at least one of phosphorylated taus are standard values.

15. The method according to any one of claims 1 to 8, wherein the subject is determined to have dementia of Alzheimer's disease when the level or amount of at least one of drebrin A or drebrin A-derived molecules is a standard value, the level or amount of at least one of phosphorylated taus is an abnormal value, and the level or amount of at least one of amyloid βs is an abnormal value.

16. The method according to any one of claims 1 to 15, wherein when the biological sample is cerebrospinal fluid, an abnormal value of the level or amount of at least one of drebrin A or drebrin A-derived molecules is about 100 pg/ml or more.

17. The method according to claim 4 or 5, wherein when the biological sample is cerebrospinal fluid, an abnormal value of the level or amount of at least one of amyloid βs is about 900 pg/ml or less.

18. The method according to claim 4 or 5, wherein when the biological sample is cerebrospinal fluid, an abnormal value of the level or amount of at least one of the phosphorylated taus is about 50 pg/ml or more.

19. The method according to any one of claims 1 to 19, wherein the level or amount of at least one of drebrin A or drebrin A-derived molecules is measured by using an antibody recognizing a drebrin A-specific epitope.

20. A method for determining a cognitive dysfunction stage of a subject, comprising:
a step of determining a cognitive dysfunction stage based on a level or amount of at least one of components of a biological sample collected from the subject,
wherein (a) the subject is determined to have mild cognitive impairment (MCI) of Alzheimer's disease when the level or amount of at least one of drebrin A or drebrin A-derived molecules is an abnormal value, in the biological sample collected from the subject,
(b) the subject is determined to be cognitively unimpaired (CU) in Alzheimer's disease when the biological sample is cerebrospinal fluid, and the level or amount of at least one of drebrin A or drebrin A-derived molecules is an abnormal value, and the level or amount of at least one of amyloid βs is a standard value, in the biological sample collected from the subject,
(c) the subject is determined to be cognitively unimpaired (CU) in Alzheimer's disease when the biological sample is cerebrospinal fluid, and the level or amount of at least one of drebrin A or drebrin A-derived molecules is a standard value, and 1) when the level or amount of at least one of phosphorylated taus is an abnormal value, or 2) when the level or amount of at least one of amyloid βs is an abnormal value, in the biological sample collected from the subject,
(d) the subject is determined to be cognitively unimpaired (CU) in Alzheimer's disease when the biological sample is cerebrospinal fluid, and the level or amount of at least one of drebrin A or drebrin A-derived molecules is a standard value, the level or amount of at least one of phosphorylated taus is an abnormal value, and the level or amount of at least one of amyloid βs is a standard value, in the biological sample collected from the subject,
(e) the subject is determined to have no brain disorder when all the levels or amounts of at least one of drebrin A or drebrin A-derived molecules, at least one of amyloid βs, and at least one of phosphorylated taus are standard values, in the biological sample collected from the subject, and
(f) the subject is determined to have dementia of Alzheimer's disease when the level or amount of at least one of drebrin A or drebrin A-derived molecules is a standard value, the level or amount of at least one of phosphorylated taus is an abnormal value, and the level or amount of at least one of amyloid βs is an abnormal value in the biological sample collected from the subject.

21. An agent for determining a cognitive dysfunction stage of a subject based on a level or amount of at least one of drebrin A or drebrin A-derived molecules, the agent comprising an antibody recognizing a drebrin A-specific epitope.

22. A method in which a level or amount of at least one of drebrin A or drebrin A-derived molecules is used as an index for determining a cognitive dysfunction stage of a subject, comprising:
a step of measuring the level or amount of at least one of drebrin A or drebrin A-derived molecules in a biological sample collected from the subject; and
a step of using the level or amount as an index for determining a cognitive dysfunction stage of the subject.
